# Europäisches Patentamt
# European Patent Office
# Office européen des brevets

(19)

(11) Veröffentlichungsnummer: **0 082 349**
**B1**

(12)

# EUROPÄISCHE PATENTSCHRIFT

(45) Veröffentlichungstag der Patentschrift:
**27.12.84**

(21) Anmeldenummer: **82110974.1**

(22) Anmeldetag: **27.11.82**

(51) Int. Cl.³: **C 07 C 51/573,** C 07 C 53/12,
C 07 C 67/54, C 07 C 69/16,
C 07 C 45/82, C 07 C 49/08

(54) **Verfahren zur Entfernung von Aceton aus Reaktionsgemischen von Carbonylierungsreaktionen.**

(30) Priorität: **11.12.81 DE 3149094**

(43) Veröffentlichungstag der Anmeldung:
**29.06.83 Patentblatt 83/26**

(45) Bekanntmachung des Hinweises auf die Patenterteilung:
**27.12.84 Patentblatt 84/52**

(84) Benannte Vertragsstaaten:
**AT BE CH DE FR GB IT LI NL**

(56) Entgegenhaltungen:
**DE - A - 2 505 471**

(73) Patentinhaber: **HOECHST AKTIENGESELLSCHAFT,**
**Postfach 80 03 20, D-6230 Frankfurt am Main 80 (DE)**

(72) Erfinder: **Erpenbach, Heinz, Dr., Oberbuschweg 22,**
**D-5000 Köln 50 (DE)**
Erfinder: **Gehrmann, Klaus, Dr.,**
**Geschwister-Scholl-Strasse 31, D-5042 Erftstadt (DE)**
Erfinder: **Hörstermann, Peter, Dr.,**
**Theodor-Heuss-Strasse 2-4, D-5042 Erftstadt (DE)**
Erfinder: **Kohl, Georg, von Geyr-Ring 61a, D-5030 Hürth**
**(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Verfahren zur Entfernung von Aceton aus Reaktionsgemischen, welche bei der Umsetzung von Methylacetat und/oder Dimethylether mit Kohlenmonoxid und ggf. Wasserstoff bei erhöhten Temperaturen zu Essigsäureanhydrid und ggf. Ethylidendiacetat in Gegenwart eines aus Carbonylkomplexen von Edelmetallen der Gruppe VIII des Periodensystems, Essigsäure, einer Organophosphor- oder Organostickstoffverbindung, Methyljodid im Molverhältnis 1:(25-600):(10-300):(10-300) und ggf. Verbindungen carbonylbildender Nichtedelmetalle bestehenden Katalysatorsystems, z.B. gemäss DE-OS Nrn. 2450965, 2836084, 2939839 und 2941232, erhalten wurden.

Bei derartigen Carbonylierungsreaktionen entstehen Reaktionsgemische, die neben den nichtflüchtigen, in erster Linie aus Verbindungen eines Edelmetalls der Gruppe VIII des Periodensystems sowie den als Promotoren verwendeten Organophosphor- bzw. Organostickstoffverbindungen bestehenden Katalysatorbestandteilen als flüchtige Komponenten Essigsäureanhydrid, Ethylidendiacetat, Essigsäure und Methyljodid sowie nichtumgesetztes Methylacetat und ggf. Dimethylether enthalten. Daneben enthält das Reaktionsgemisch geringe Mengen Aceton, das als unerwünschtes Nebenprodukt der Carbonylierung auftritt. In kontinuierlich betriebenen Carbonylierunganlagen erfolgt in der Regel zunächst eine Abtrennung des nichtflüchtigen Katalysatorsystems von den flüchtigen Bestandteilen. Die flüchtigen Komponenten werden dann weiter destillativ aufgetrennt, wobei zuerst die Leichtsieder Methyljodid und nichtumgesetztes Methylacetat bzw. Dimethylether über Kopf abdestilliert werden.

Aufgrund der geringen Siedepunktdifferenz des Acetons zum Methylacetat (Methylacetat: 57° C, Aceton: 56° C) enthält der so abdestillierte Leichtsiederanteil auch das als Nebenprodukt entstandene Aceton. Da die abdestillierten Leichtsieder vollständig zum Reaktor zurückgeführt werden, muss es im Laufe der Zeit zwangsläufig zu einer Anreicherung des Acetons im Reaktionsprodukt bzw. Leichtsiedergemisch kommen. Es hat sich jedoch gezeigt, dass höhere Acetonkonzentrationen während der Reaktion sowohl die Aktivität des Katalysators als auch die Bildung unerwünschter Nebenprodukte ungünstig beeinflussen.

Die DE-OS Nr. 2952516 (= US-PS Nr. 4252748) beschreibt bereits ein Verfahren zur Abtrennung von Aceton aus den flüchtigen Bestandteilen des Reaktionsgemisches, das bei der Umsetzung von Methylacetat mit Kohlenmonoxid und Wasserstoff in Gegenwart eines Edelmetalls der Gruppe VIII des Periodensystems und von Methyljodid erzeugt wird, welches dadurch gekennzeichnet ist, dass man in dem Gemisch der flüchtigen Bestandteile durch Einführung von Aceton in den Carbonylierungsbereich ein Molverhältnis von Aceton zu Methyljodid von wenigstens 1:10 einstellt, dieses Gemisch aus flüchtigen Bestandteilen einer fraktionierten Destillation zur Abtrennung praktisch des gesamten Methyljodids und eines Teils des Acetons und Methylacetats als Destillat unterwirft, wobei die Menge an abgetrenntem Aceton praktisch der in die Reaktion eingeführten Menge entspricht, dass man das übrige Aceton und Methylacetat aus dem Rückstand dieser Destillation abdestilliert und danach das Aceton aus der Methylacetat/Aceton-Mischung abtrennt.

Die Isolierung des bei der Reaktion entstandenen Acetons aus dem Aceton/Methylacetat-Gemisch erfolgt über eine azeotrope Destillation mit $C_5$-Kohlenwasserstoffen gemäss US-PS Nr. 2704271, anschliessende Extraktion des Aceton/$C_5$-Kohlenwasserstoff-Gemisches mit Wasser und Fraktionierung des Acetons aus der Wasserphase.

Dieses Verfahren der Acetonabtrennung verlangt hohe Investitionen und erfordert durch den grossen Destillationsaufwand einen hohen Energiebedarf.

Es ist daher Ziel der vorliegenden Erfindung, ein einfacheres Verfahren zu finden, das eine Anreicherung des Acetons im Reaktionsgemisch von Carbonylierungsreaktionen vermeidet.

Überraschenderweise wurde gefunden, dass unter bestimmten Reaktionsbedingungen die Konzentration des Acetons im abgetrennten Leichtsiedergemisch nur bis zu einem Wert von etwa 5 Gew.-% ansteigt und dann stagniert. Das jeweils neu entstehende Aceton reagiert (Kondensiert) unter den gewählten Reaktions- und Aufarbeitungsbedingungen zu Folgeprodukten mit einer C-Zahl von 6 bis 12. Diese Folgeprodukte besitzen Siedepunkte, die einerseits eine destillative Abtrennung vom nichtflüchtigen Katalysatorsystem gemeinsam mit den anderen destillierbaren Anteilen gestatten, andererseits aber bei der anschliessenden Abtrennung der Leichtsieder Methyljodid und Methylacetat aus den flüchtigen Bestandteilen zusammen mit der Essigsäure und dem gebildeten Essigsäureanhydrid im Sumpfprodukt dieser Trennstufe verbleiben. Auf diese Weise wird erreicht, dass die Acetonfolgeprodukte, die die Katalysatoraktivität stark hemmen und in der Reaktionszone zu weiteren Kondensationsreaktionen führen, weder mit dem nichtflüchtigen Teil des Katalysatorsystems noch mit den Leichtsiedern Methyljodid und Methylacetat zum Reaktor zurückgeführt werden. Eine Anreicherung dieser Produkte im Reaktor ist auf diese Weise ausgeschlossen. Vielmehr bleiben die Acetonkondensationsprodukte nach destillativer Abtrennung von Essigsäure und Essigsäureanhydrid im Sumpf der Anhydridkolonne zurück und können dort ausgeschleust werden.

Die Kondensation des Acetons hängt überwiegend vom Mengenverhältnis der Reaktionsteilnehmer in der Reaktionszone und in der Stufe der Katalysatorabtrennung ab. Es wurde gefunden, dass bei einem molaren Verhältnis Edelmetall zu Essigsäure zu Organostickstoff- bzw. Organophosphorverbindung zu Methyljodid von 1:(25-500):(10-100):(15-150) und einem stationären Acetongehalt von 5 Gew.-% im Leichtsiedergemisch die kondensation des während der Reaktion jeweils neu entstehenden Acetons vollständig abläuft. Geht man von geringeren Acetongehalten aus, so erfolgt zunächst eine Anreicherung des Acetons bis zu einem Wert von 5 Gew.-% im Leichtsiedergemisch, bevor eine nennenswerte Bildung von Kondensationsprodukten zu beobachten ist. Setzt man dagegen Leichtsiedergemische mit mehr als 5 Gew.-% Aceton in die Reaktionszone ein, so wird der Acetongehalt unter verstärker Bildung von Kondensationsprodukten wieder auf einen Gehalt von 5 Gew.-% Aceton im Leichtsiedergemisch abgebaut und bleibt dann bei diesem Wert.

Im einzelnen ist das Verfahren der Erfindung nunmehr dadurch gekennzeichnet, dass man das bei der Umsetzung als Nebenprodukt anfallende Aceton bei Temperaturen von 50 bis 250° C, Drukken von 0,01 bis 150 bar und einem wie oben definierten Molverhältnis von 1:(25-500):(10-100):(15-150) überwiegend zu höhersiedenden Folgeprodukten kondensiert, diese zusammen mit den flüchtigen Bestandteilen des Reaktionsgemisches von den nichtflüchtigen Bestandteilen des Katalysatorsystems abdestilliert und das Destillat in einer nachfolgenden Destillationszone in ein aus Methyljodid, nichtumgesetztem Methylacetat und/oder Dimethylether sowie restlichem Aceton bestehendes Leichtsiedergemisch und ein aus Essigsäure, Essigsäureanhydrid, ggf. Ethylidendiacetat und den Acetonfolgeprodukten bestehendes Sumpfprodukt auftrennt.

Darüber hinaus kann das Verfahren der Erfindung wahlweise und bevorzugt dadurch gekennzeichnet sein, dass man

a) die destillative Abtrennung der flüchtigen Bestandteile des Reaktionsgemisches von den nichtflüchtigen Bestandteilen des Katalysatorsystems bei Temperaturen von 50 bis 170° C und Drucken von 0,01 bis 3 bar vornimmt;

b) die destillative Abtrennung der flüchtigen Bestandteile des Reaktionsgemisches von den nichtflüchtigen Bestandteilen des Katalysatorsystems in Gegenwart von Kohlenmonoxid und ggf. Wasserstoff vornimmt;

c) aus dem Sumpfprodukt der Destillationszone nacheinander Essigsäure, Essigsäureanhydrid und gf. Ethylidendiacetat abdestilliert.

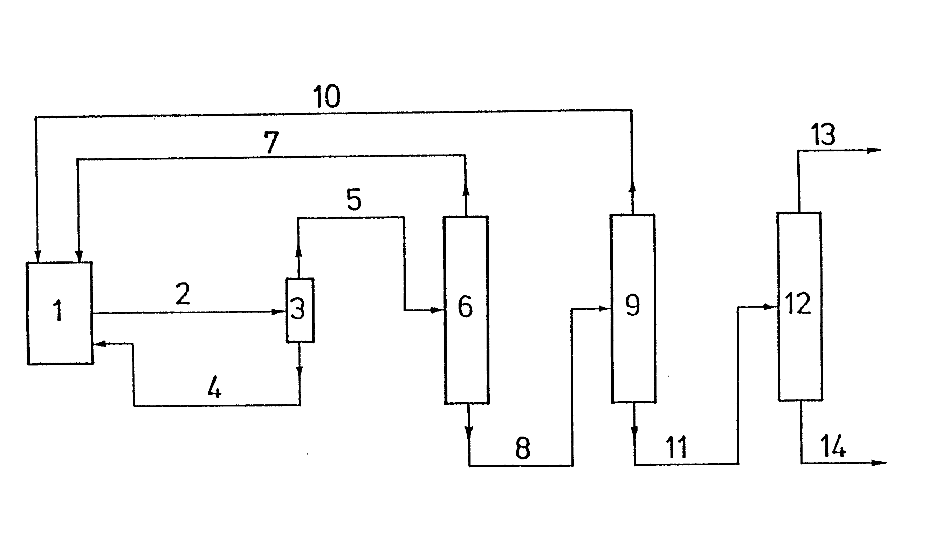

Die Erfindung soll anhand des Fliessschemas der Zeichnung näher erläutert werden:

In einem Carbonylierungsreaktor 1 werden Methylacetat und/oder Dimethylether mit Kohlenmonoxid und gff. Wasserstoff in Gegenwart eines Katalysatorsystems aus Carbonylkomplexen von Edelmetallen der Gruppe VIII des Periodensystems, Essigsäure, einer Organophosphor- oder Organostickstoffverbindung und Methyljodid im Molverhältnis 1:(25-500):(10-100):(15-150) bei Temperaturen von 150 bis 200° C und Drucken von 25 bis 150 bar umgesetzt. Das Reaktionsgemisch gelangt über Leitung 2 in die Katalysatorabtrennung 3, wo die destillierbaren Anteile unter einem Druck von 0,1 bis 2 bar und bei Temperaturen von 75 bis 170° C vom nichtflüchtigen Katalysatorsystem abgetrennt werden. Das Katalysatorsystem wird über Leitung 4 zum Reaktor zurückgeführt. Die flüchtigen Bestandteile gelangen über Leitung 5 in die erste Destillationsstufe 6, in der die Leichtsieder Methyljodid, nichtumgesetztes Methylacetat und Aceton über Kopf abgetrennt werden. Dieses Kopfprodukt wird über Leitung 7 zum Reaktor 1 zurückgeführt. Das als Sumpfprodukt zurückbleibende Gemisch aus Essigsäure, Essigsäureanhydrid und Ethylidendiacetat, das auch die hochsiedenden Kondensationsprodukte des Acetons enthält, wird über Leitung 8 in die zweite Destillationsstufe 9 gefördert, wo über Kopf die Essigsäure abgetrennt und über Leitung 10 zum Reaktor 1 zurückgeführt wird. Das Sumpfprodukt gelangt dann über Leitung 11 in die dritte Destillationsstufe 12, in der das gebildete Essigsäureanhydrid über Leitung 13 als Kopfprodukt erhalten wird. Als Sumpfprodukt bleiben Ethylidendiacetat und die Kondensationsprodukte des Acetons zurück und werden über Leitung 14 ausgeschleust. Eine nachfolgende destillative Abtrennung (nicht gezeichnet) des Ethylidendiacetats (Sdp. 169° C bei 1013 mbar; 111° C bei 150 mbar) ist möglich.

Der Vorteil dieses Verfahrens besteht darin, dass sich das als unerwünschtes Nebenprodukt entstehende Aceton nur bis zu einer Konzentration von 5 Gew.-% im Leichtsiedergemisch anreichert. Das darüber hinaus gebildete Aceton kondensiert unter den erfindungsgemässen Bedingungen zu Folgeprodukten, die ohne zusätzlichen Destillationsaufwand über den Sumpf der dritten Destillationsstufe 12 aus dem System entfernt werden können.

*Beispiel 1:*

Die Carbonylierung erfolgt bei einer Temperatur von 185° C und einem CO-Partialdruck von 50 bar. Der Gesamtdruck im Reaktor 1 steigt bis auf 70 bar. Das Reaktionsgemisch enthält Rh-Komplex, Essigsäure, Methyltributylphosphoniumjodid, Methyljodid und Methylacetat im Molverhältnis 1:152:37:68:340. Dem Reaktor 1 werden 23 450 g/h Reaktionsgemisch entnommen und in der Katalysatorabtrennung 3 bei 150 mbar Druck und 95° C unter Zudosierung von 20 l/h Synthesegas (CO/$H_2$ = 1:1) in 6900 g/h Katalysatorsystem und 16 550 g/h flüchtige Bestandteile zerlegt. Die flüchtigen Anteile werden in der ersten Destillationsstufe 6 in 6137 g/h eines Gemisches aus Essigsäure und Essigsäureanhydrid als Sumpfprodukt und 10 413 g/h der über Kopf abdestillierten Leichtsiederfraktion aus Methyljodid und Methylacetat getrennt. Katalysatorsystem und Leichtsiederfraktion werden über die Leitungen 4 bzw. 7 zum Reaktor 1 zurückgeleitet.

Zu Beginn des Versuches ist das Leichtsiedergemisch frei von Aceton. Der zeitliche Verlauf der Acetonkonzentration in diesem Gemisch ergibt sich aus nachfolgender Tabelle.

Aus dem Sumpfprodukt der ersten Destillationsstufe 6 werden 2636 g/h Essigsäure in der zweiten Destillationsstufe 9 abdestilliert und über Leitung 10 zum Reaktor zurückgeführt. Im Sumpf (3501 g/h) verbleiben Essigsäureanhydrid neben geringen Mengen Ethylidendiacetat und den Kondensationsprodukten des Acetons. Die letzte Destillation in der dritten Destillationsstufe 12 liefert 3450 g/h Essigsäureanhydrid als Kopfprodukt und 51 g/h eines Rückstandes, der neben wenig Essigsäureanhydrid das Ethylidendiacetat und die Kondensationsprodukte des Acetons enthält. Zu Beginn des Versuches werden in diesem Sumpfprodukt noch keine Kondensationsprodukte nachgewiesen. Der zeitliche Verlauf der Konzentration dieser Acetonfolgeprodukte im Sumpf der dritten Destillationsstufe 12 ergibt sich ebenfalls aus der nachfolgenden Tabelle.

| Zeit (d) | Aceton-Konzentration in Strom 7 (Gew.-%) | Konzentration der Acetonfolgeprodukte im Strom 14 (Gew.-%) |
|---|---|---|
| 0 | 0 | 0 |
| 10 | 2,0 | 0,02 |
| 20 | 3,8 | 0,2 |
| 30 | 4,5 | 1,1 |
| 40 | 5,0 | 2,4 |
| 50 | 5,0 | 2,4 |
| 60 | 5,0 | 2,4 |

*Beispiel 2:*

Die Versuchsdurchführung entspricht der des Beispiels 1. Zu Beginn des Versuches wird jedoch die Konzentration des Acetons im Leichtsiedergemisch durch entsprechenden Zusatz von Aceton auf 9 Gew.-% eingestellt. Der zeitliche Verlauf sowohl der Acetonkonzentration im Leichtsiedergemisch als auch der Konzentration der Acetonkondensationsprodukte im Sumpf der dritten Destillationsstufe 12 ergibt sich aus nachfolgender Tabelle.

| Zeit (d) | Aceton-Konzentration in Strom 7 (Gew.-%) | Konzentration der Acetonfolgeprodukte im Strom 14 (Gew.-%) |
|---|---|---|
| 0 | 9,0 | — |
| 10 | 6,5 | 4,5 |
| 20 | 5,3 | 3,2 |
| 30 | 5,0 | 2,4 |
| 40 | 5,0 | 2,4 |
| 50 | 5,0 | 2,4 |

*Beispiel 3:*

Die Versuchsdurchführung entspricht der im Beispiel 1. Lediglich in der Katalysatorabtrennung 3 ist der Druck auf 1,2 bar und die Temperatur auf 145° C erhöht. Zu Beginn des Versuches wird durch entsprechenden Zusatz eine Acetonkonzentration im Leichtsiedergemisch von 5 Gew.-% eingestellt. Der zeitliche Verlauf der Acetonkonzentration im Leichtsiedergemisch und der Konzentration der Acetonfolgeprodukte im Sumpf der dritten Destillationsstufe 12 ergibt sich aus nachfolgender Tabelle.

| Zeit (d) | Aceton-Konzentration in Strom 7 (Gew.-%) | Konzentration der Acetonfolgeprodukte im Strom 14 (Gew.-%) |
|---|---|---|
| 0 | 5,0 | — |
| 10 | 5,0 | 2,4 |
| 20 | 5,0 | 2,4 |
| 30 | 5,0 | 2,4 |

*Beispiel 4:*

Die Carbonylierung erfolgt bei einer Temperatur von 185° C und einem Gesamtdruck von 110 bar. Anstelle des in den vorhergehenden Beispielen verwendeten Methylacetats wird Dimethylether als Ausgangsmaterial eingesetzt. Das Reaktionsgemisch enthält Rh-Komplex, Essigsäure, Methyltributylphosphoniumjodid, Methyljodid, Methylacetat und Dimethylether im Molverhältnis 1:152:37:68:287:53. Dem Reaktor 1 werden 23 149 g/h Reaktionsgemisch entnommen und in der Katalysatorabtrennung 3 bis 150 mbar Druck und 95° C unter Zudosierung von 20 l/h Synthesegas (CO:$H_2$ = 1:1) in 6900 g/h Katalysatorsystem und 16 249 g/h flüchtige Bestandteile zerlegt. Die flüchtigen Anteile werden in der ersten Destillationsstufe 6 in 5836 g/h eines Gemisches aus Essigsäure und Essigsäureanhydrid als Sumpfprodukt und 10 413 g/h der über Kopf abdestillierten Leichtsiederfraktion aus Methyljodid, Methylacetat und Dimethylether getrennt. Katalysatorsystem und Leichtsiederfraktion werden über die Leitungen 4 bzw. 7 zum Reaktor 1 zurückgeleitet.

Zu Beginn des Versuches ist das Leichtsiedergemisch frei von Aceton. Der zeitliche Verlauf der Acetonkonzentration in diesem Gemisch ergibt sich aus nachfolgender Tabelle.

Aus dem Sumpfprodukt der ersten Destillationsstufe 6 werden 2636 g/h Essigsäure in der zweiten Destillationsstufe 9 abdestilliert und über Leitung 10 zum Reaktor zurückgeführt. Im Sumpf (3200 g/h) verbleiben Essigsäureanhydrid neben geringen Mengen Ethylidendiacetat und den Kondensationsprodukten des Acetons. Die letzte Destillation in der dritten Destillationsstufe 12 liefert 3150 g/h Essigsäureanhydrid als Kopfprodukt und 50 g/h eines Rückstandes, der neben wenig Essigsäureanhydrid das Ethylidendiacetat und die Kondensationsprodukte des Acetons enthält. Zu Beginn des Versuches werden in diesem Sumpfprodukt noch keine Kondensationsprodukte nachgewiesen. Der zeitliche Verlauf der Konzentration dieser Acetonfolgeprodukte im Sumpf der dritten Destillationsstufe 12 ergibt sich ebenfalls aus der nachfolgenden Tabelle.

| Zeit (d) | Aceton-Konzentration in Strom 7 (Gew.-%) | Konzentration der Acetonfolge- produkte im Strom 14 (Gew.-%) |
|---|---|---|
| 0 | 0 | 0 |
| 10 | 1,9 | 0,02 |
| 20 | 3,7 | 0,2 |
| 30 | 4,5 | 1,0 . |
| 40 | 5,0 | 2,4 |
| 50 | 5,0 | 2,4 |

## Patentansprüche

1. Verfahren zur Entfernung von Aceton aus Reaktionsgemischen, welche bei der Umsetzung von Methylacetat und/oder Dimethylether mit Kohlenmonoxid und ggf. Wasserstoff bei erhöhten Temperaturen zu Essigsäureanhydrid und ggf. Ethylidendiacetat in Gegenwart eines aus Carbonylkomplexen von Edelmetallen der Gruppe VIII des Periodensystems, Essigsäure, einer Organophosphor- oder Organostickstoffverbindung, Methyljodid im Molverhältnis 1:(25-600):(10-300):(10-300) und ggf. Verbindungen carbonylbildender Nichtedelmetalle bestehenden Katalysatorsystems erhalten wurden, dadurch gekennzeichnet, dass man das bei der Umsetzung als Nebenprodukt anfallende Aceton bei Temperaturen von 50 bis 250° C, Drucken von 0,01 bis 150 bar und einem wie oben definierten Molverhältnis von 1:(25-500):(10-100):(15-150) überwiegend zu höhersiedenden Folgeprodukten kondensiert, diese zusammen mit den flüchtigen Bestandteilen des Reaktionsgemisches von den nichtflüchtigen Bestandteilen des Katalysatorsystems abdestilliert und das Destillat in einer nachfolgenden Destillationszone in ein aus Methyljodid, nichtumgesetztem Methylacetat und/oder Dimethylether sowie restlichem Aceton bestehendes Leichtsiedergemisch und ein aus Essigsäure, Essigsäureanhydrid, ggf. Ethylidendiacetat und den Acetonfolgeprodukten bestehendes Sumpfprodukt auftrennt.

2. Verfahren nach Anspruch 1, dadurch gekennzeichnet, dass man die destillative Abtrennung der flüchtigen Bestandteile des Reaktionsgemisches von den nichtflüchtigen Bestandteilen des Katalysatorsystems bei Temperaturen von 50 bis 170° C und Drucken von 0,01 bis 3 bar vornimmt.

3. Verfahren nach einem der Ansprüche 1 oder 2, dadurch gekennzeichnet, dass man die destillative Abtrennung der flüchtigen Bestandteile des Reaktionsgemisches von den nichtflüchtigen Bestandteilen des Katalysatorsystems in Gegenwart von Kohlenmonoxid und ggf. Wasserstoff vornimmt.

4. Verfahren nach einem der Ansprüche 1 bis 3, dadurch gekennzeichnet, dass man aus dem Sumpfprodukt der Destillationszone nacheinander Essigsäure, Essigsäureanhydrid und ggf. Ethylidendiacetat abdestilliert.

## Claims

1. Process for removing acetone from reaction mixtures obtained by reacting methyl acetate and/or dimethylether with carbon monoxide and optionally hydrogen at elevated temperatures to give acetic anhydride and optionally ethylidene diacetate in the presence of a catalyst system consisting of carbonyl complexes of noble metals belonging to Group VIII of the Periodic System of the elements, acetic acid, an organophosphorus or organonitrogen compound, and methyl iodide in a molar ratio of 1:(25-600):(10-300):(10-300) and optionally compounds of carbonyl-yielding non-noble metals, which comprises: subjecting the acetone obtained as a by-product during the reaction to condensation at temperatures of 50 to 250° C, under pressures of 0.01 to 150 bar and at a molar ratio as above defined of 1:(25-500):(10-100):(15-150) principally to higher-boiling secondary products, distillatively separating these latter together with volatile constituents of the reaction mixture from the non-volatile constituents of the catalyst system, and separating the distillate in a subsequent distilling zone into a low boiler mixture consisting of methyl iodide, unreacted methyl acetate and/or dimethylether and residual acetone, and into a base product consisting of acetic acid, acetic anhydride and optionally ethylidene diacetate and the acetone secondary products.

2. Process as claimed in Claim 1, wherein the volatile constituents of the reaction mixture are distillatively separated from the non-volatile constituents of the catalyst system at temperatures of 50 to 170° C under pressures of 0.01 to 3 bar.

3. Process as claimed in Claim 1 or 2, wherein the volatile constituents of the reaction mixture are distillatively separated from the non-volatile constituents of the catalyst system in the presence of carbon monoxide and optionally hydrogen.

4. Process as claimed in any of Claims 1 to 3, wherein the base product coming from the distilling zone is successively freed distillatively from acetic acid, acetic anhydride and optionally ethylidene diacetate.

## Revendications

1. Procédé pour éliminer l'acétone de mélanges de réaction obtenus par réaction de l'acétone de méthyle et/ou de l'éther diméthylique avec le monoxyde de carbone et, éventuellement, l'hydrogène, à température élevée avec formation d'anhydride acétique et, éventuellement, de diacétate d'éthylidène en présence d'un système catalytique constitué par des complexes carbonylés de métaux nobles appartenant au groupe VIII de la Classification périodique, de l'acide acétique, un composé organophosphoré ou organoazoté, de l'iodure de méthyle dans un rapport molaire de 1:(25-600):(10-300):(10-300) et, éventuellement, des composés de métaux non nobles formant des carbonyles, caractérisé en ce que l'on condense l'acétone obtenue en cours de réaction comme sous-produit à des températures de 50 à 250° C sous des pressions de 0,01 à 150 bar et dans un rapport molaire comme défini ci-dessus de 1:(25-500):(10-100):(15-150) principalement en dérivés à points d'ébullition plus élevés, en ce qu'on sépare par distillation ces derniers conjointement avec les constituants volatils du mélange réactionnel des constituants non volatils du système catalytique et en ce qu'on sépare le distillat, dans une zone de distillation placée à la suite, en un mélange de composés à bas point d'ébullition constitué par de l'iodure de méthyle, de l'acétate de méthyle et/ou de l'éther diméthylique n'ayant pas réagi et de l'acétone résiduaire, et en un produit de queue constitué par de l'acide acétique, de l'anhydride acétique, éventuellement du diacétate d'éthylidène, et par les dérivés d'acétone.

2. Procédé selon la revendication 1, caractérisé en ce que l'on effectue la séparation par distillation des constituants volatils du mélange de réaction et des constituants non volatils du système catalytique à des températures de 50 à 170° C et sous des pressions de 0,01 à 3 bar.

3. Procédé selon l'une des revendications 1 ou 2, caractérisé en ce que l'on effectue la séparation par distillation des constituants volatils du mélange de réaction et des constituants non volatils du système catalytique en présence de monoxyde de carbone et, éventuellement, d'hydrogène.

4. Procédé selon l'une des revendications 1 à 3, caractérisé en ce que l'on sépare successivement par distillation l'acide acétique, l'anhydride acétique et, éventuellement, le diacétate d'éthylidène du produit de queue de la zone de distillation.

0 082 349